## Europäisches Patentamt

## European Patent Office

## Office européen des brevets

(11) Veröffentlichungsnummer: **0 232 486**
**B1**

(12) # EUROPÄISCHE PATENTSCHRIFT

(45) Veröffentlichungstag der Patentschrift:
**06.09.89**

(51) Int. Cl.⁴: **B01D 13/02**, C12P 13/04,
C07C 99/12

(21) Anmeldenummer: **86116353.3**

(22) Anmeldetag: **25.11.86**

(54) **Verfahren zur Aufarbeitung der nach Abtrennung des Enzyms verbleibenden Lösung aus der enzymatischen Racematspaltung einer N-Acetyl-DL-aminocarbonsäure in Gegenwart einer L-Aminosäureacylase.**

(30) Priorität: **08.02.86 DE 3603986**

(43) Veröffentlichungstag der Anmeldung:
**19.08.87 Patentblatt 87/34**

(45) Bekanntmachung des Hinweises auf die Patenterteilung:
**06.09.89 Patentblatt 89/36**

(84) Benannte Vertragsstaaten:
**BE DE ES FR GB LU SE**

(56) Entgegenhaltungen:
**EP-A- 0 156 513**
**DE-A- 2 907 450**
**US-A- 3 231 485**
**US-A- 3 330 749**

**CHEMIE-INGENIEUR-TECHNIK, Band 56, Nr. 3, März 1984, Seiten 214-220, Nürnberg, DE; H. STRATHMANN et al.: "Die Elektrodialyse - ein Membranverfahren mit vielen Anwendungsmöglichkeiten"**

(73) Patentinhaber: **Degussa Aktiengesellschaft, Weissfrauenstrasse 9, D-6000 Frankfurt am Main 1(DE)**

(72) Erfinder: **Koberstein, Edgar, Dr., Wolfskernstrasse 8, D-8755 Alzenau(DE)**
Erfinder: **Lehmann, Thomas, Dr., Bergerstrasse 338, D-6000 Frankfurt Am Main 60(DE)**

**Beschreibung**

Die Erfindung betrifft ein Verfahren zur Aufarbeitung der nach Abtrennung des Enzyms Verbleibenden Lösung aus der enzymatischen Racematspaltung einer N-Acetyl-DL-aminocarbonsäure in Gegenwart einer L-Aminosäureacylase durch Elektrodialyse.

Bei der synthetischen Herstellung von enantiomerenreinen L-Aminosäuren wird in vielen Fällen der Umweg beschritten, daß man das zunächst erhaltene Racemat acetyliert und dann die racemische N-Acetyl-DL-aminosäure einer Spaltung durch eine L-Aminosäureacylase unterwirft. Nach Abtrennung des Enzyms wird die durch Hydrolyse freigesetzte L-Aminosäure in geeigneter Weise isoliert. Die nicht hydrolysierte N-Acetyl-D(L)-aminosäure kann nach Reracemisierung erneut der Racematspaltung unterworfen werden.

Die nach Abtrennung des Enzyms verbleibenden Lösungen aus der enzymatischen Racematspaltung enthalten beispielsweise 0,15 bis 0,7 Mol/l L-Aminosäure, 0,15 bis 0,9 Mol/l N-Acetylderivate (hauptsächlich N-Acetyl-D-aminosäure und eine geringere Menge N-Acetyl-L-aminosäure) und 0,15 bis 0,7 Mol/l Essigsäure, jeweils in Form eines Alkalimetallsalzes, vorzugsweise eines Natriumsalzes, und geringe Mengen an Effektorsalzen, beispielsweise an $CoCl_2$.

Diese Lösung wird üblicherweise dadurch entsalzend aufgearbeitet, daß man sie über eine mit stark saurem Kationenaustauschermaterial gefüllte Ionenaustauschersäule gibt. Je nach Betriebsweise erhält man eine mehr oder weniger entsalzte Aminosäure-Lösung, eine stark saure Fraktion, die die Anionen als freie Säuren enthält und außerdem erhebliche Mengen von verbrauchten Regenerationslösungen, die die ausgetauschten Kationen enthalten und in der Regel als Abwasser entsorgt werden müssen. Bei schlecht oder nur mäßig löslichen Aminosäuren treten zusätzliche Probleme dadurch auf, daß sie als freie Säuren in der Ionenaustauschersäule ausfallen können und diese zu verstopfen drohen. Dem wird in der Regel durch Temperaturerhöhung begegnet, was wiederum Nachteile bei Vorliegen thermisch labiler Verbindungen verursacht.

Aus der DE-OS 29 07 450 ist bereits ein Verfahren zur Aufarbeitung solcher Lösungen durch Elektrodialyse bekannt. Das bekannte Verfahren wird in einer Elektrodialysevorrichtung durchgeführt, die aus einer Vielzahl von Kammern besteht, die abwechselnd durch Kationen- und Anionenaustauschermembranen voneinander getrennt sind, welche ihrerseits zwischen einem einzigen Elektrodenpaar angeordnet sind. Abgesehen von der Anoden- und der Kathodenzelle werden die Kammern so betrieben, daß immer abwechselnd eine Kammer als Speise- und Diluatkammer und die benachbarte Kammer als Konzentratkammer dient. In den Speise- und Diluatkammern wird die aufzuarbeitende Lösung umgewälzt, während der Elektrodialyse können die in der Lösung enthaltenen Anionen und Kationen durch die Anionenaustauschermembran bzw. die Kationenaustauschermembran hindurchtreten in die jeweils benachbarte Konzentratkammer. Die amphotere Aminosäure wird in der Speise- und Diluatkammer dadurch festgehalten, daß die Elektrodialysevorrichtung mit einer hohen Stromdichte nahe der Grenzstromdichte betrieben wird. Dabei wird an den Membranen eine pH-Schranke aufgebaut, welche die amphotere Aminosäure nicht durch die Membranen hindurchtreten läßt. Bei dem bekannten Verfahren können zwar enantiomerenreine Aminosäuren in guter Ausbeute in Form ihrer wäßrigen Lösungen aus den Diluatkammern abgezogen werden, nachteilig ist aber, daß sich die ursprünglich enthaltenen Anionen und Kationen wieder vermischen können und infolgedessen aus den Konzentratkammern nur wäßrige Salzlösungen abgezogen werden können.

Das erfindungsgemäße Verfahren ist nun dadurch gekennzeichnet, daß die verwendete Elektrodialysevorrichtung aus einer Vielzahl von 3-Kammer-Paketen besteht, die ihrerseits jeweils eine Speise- und Diluatkammer (1), eine kathodenseitig angeordnete Konzentratkammer (2) und eine anodenseitig angeordnete Konzentratkammer (3) aufweisen, wobei jedes 3-Kammer-Paket von jedem der beiden benachbarten durch eine Einrichtung (4) getrennt ist, in der Wasser in $OH^-$-Ionen und $H^+$-Ionen zerlegt wird, und daß nach beendeter Elektrodialyse aus den Diluatkammern (1) eine wäßrige Lösung der L-Aminosäure und von höchstens 50% der ursprünglich vorhandenen Anionen in Form ihrer Salze, aus den kathodenseitig angeordneten Konzentratkammern (2) eine wäßrige Lösung der ursprünglich vorhandenen Kationen als Hydroxide und aus den anodenseitig angeordneten Konzentratkammern (3) eine wäßrige Lösung von N-Acetyl-D(L)-aminosäure und Essigsäure in Form der freien Säuren abgezogen wird.

Die beim erfindungsgemäßen Verfahren vorgesehenen Einrichtungen (4) zur Wasserzerlegung können aus einer zusätzlichen Kammer bestehen, die mit Wasser gefüllt ist, das einen Leitfähigkeitsvermittler enthält, für welchen die die Kammer begrenzenden Membranen undurchlässig sind. Geeignete Leitfähigkeitsvermittler sind suspendierte inerte leitfähige Materialien, wie Aktivkohle, Ruß oder sphärische Metallpartikelchen, oder höhermolekulare Säuren, wie Polyacrylsäuren oder Polystyrolsulfonsäuren, oder Basen, beispielsweise gegebenenfalls substituierte Polyethylenimine.

Alternativ können die Einrichtungen (4) zur Wasserzerlegung auch aus sogenannten bipolaren Membranen bestehen. Diese sind aufgebaut aus je einer Kationen- und Anionenaustauschermembran, die durch eine dünne neutrale hydrophile Schicht voneinander getrennt werden. Diese Struktur kann aus einem homogenen Blockpolymerisat oder aus drei aufeinander geklebten Polymerisatfolien bestehen. Dem Fachmann sind leicht ähnliche Anordnungen zugänglich, denen jedoch allen der gleiche Grundgedanke zugrunde liegt, nämlich die Zerlegung von Wassermolekülen im elektrischen Feld und die Versorgung der benachbarten Konzentratkammern (2, 3) mit den erforderlichen Ionen.

In der beim erfindungsgemäßen Verfahren zu verwendenden Elektrodialysevorrichtung sind jeder Speise- und Diluatkammer (1) zwei verschiede-

ne Konzentratkammern zugeordnet, so daß in die benachbarte kathodenseitig angeordnete Konzentratkammer (2) nur die ursprünglich vorhandenen Kationen auswandern können, die von aus der benachbarten Einrichtung (4) zur Wasserzerlegung gelieferten OH⁻-Ionen zu Hydroxiden ergänzt werden, und daß in die benachbarte anodenseitig angeordnete Konzentratkammer (3) nur die ursprünglich vorhandenen Anionen auswandern können, die von aus der anderen benachbarten Einrichtung (4) zur Wasserzerlegung gelieferten H⁺-Ionen zu den freien Säuren ergänzt werden.

Im übrigen ist die beim erfindungsgemäßen Verfahren zu verwendende Elektrodialysevorrichtung in üblicher Weise aufgebaut: Die 3-Kammer-Pakete und die jeweiligen sie trennenden Einrichtungen (4) zur Wasserzerlegung sind zwischen nur einem Elektrodenpaar angeordnet. Alle Einzelkammern sind durch je eine Anionen- und Kationenaustauschermembran und einen Dichtrahmen begrenzt und enthalten gegebenenfalls ein Stützgitter. Auf der Anodenseite der Elektrodialysevorrichtung ist zunächst eine Anodenkammer vorgesehen, die durch eine Kationenaustauschermembran begrenzt wird. Alle folgenden Kammern sind jeweils so eingebaut, daß sich Anionenaustauschermembranen und Kationenaustauschermembranen in regelmäßigen Wechsel folgen. Im Falle der Verwendung von sogenannten bipolaren Membranen als Einrichtungen (4) zur Wasserzerlegung werden diese so eingebaut, daß sich die anionenaustauschende Seite und die kationenaustauschende Seite in diesen Wechsel zwanglos einfügen. Die kathodenseitig angeordnete Konzentratkammer (2) des letzten 3-Kammer-Pakets dient gleichzeitig als Kathodenkammer.

Der Vorteil des erfindungsgemäßen Verfahrens liegt insbesondere darin, daß nach beendeter Elektrodialyse drei verschieden zusammengesetzte wäßrige Lösungen abgezogen werden können. Dadurch wird der Spielraum für die weitere Verarbeitung beträchtlich erweitert. Als erste Lösung kann aus der Speise- und Diluatkammern (1) eine wäßrige Lösung abgezogen werden, die entweder praktisch nur noch die L-Aminosäure enthält oder doch zumindest soweit entsalzt ist, daß sich aus ihr nach einer Aufkonzentrierung die reine L-Aminosäure kristallisieren läßt. Die nach der Abtrennung der auskristallisierten L-Aminosäure verbleibende Mutterlauge kann recyclet und zusammen mit frischer Lösung aus der Racematspaltung erneut der Elektrodialyse unterworfen werden. Hier fällt also praktisch kein zu entsorgendes Abwasser an. Als zweite Lösung kann aus den kathodenseitig angeordneten Konzentratkammern (2) eine wäßrige Lösung der ursprünglich vorhandenen Kationen in Form ihrer Hydroxide abgezogen werden. Diese Lösung kann ebenfalls vollständig recyclet werden und dient dazu, frische N-Acetyl-DL-aminosäure aufzulösen und den erforderlichen pH-Wert für die enzymatische Spaltung einzustellen. Auch hier fällt praktisch kein zu entsorgendes Abwasser an. Als dritte Lösung kann schließlich aus den anodenseitig angeordneten Konzentratkammern (3) eine wäßrige Lösung von N-Acetyl-D(L)-aminosäure und Essigsäure abgezogen werden, die keinerlei Kationen außer H⁺-Ionen enthält und infolgesessen nach Abdestillieren der Essigsäure und gegebenenfalls des Wassers nach beliebigen Verfahren reracemisiert und ebenfalls vollständig recyclet werden kann. Unter Umständen kann diese dritte Lösung alternativ auch zur Gewinnung des D-Enantiomeren dienen.

Das erfindungsgemäße Verfahren ist demnach wegen der allenfalls in geringer Menge anfallenden zu entsorgenden Abwässer, wegen seiner schonenden Betriebsbedingungen - die Temperatur in der Elektrodialysevorrichtung steigt kaum über 30 °C an -, wegen der erreichbaren hohen Reinheit der kristallisierten L-Aminosäure und wegen allenfalls geringer Materialverluste allen bekannten Aufarbeitungsverfahren für Lösungen aus der Racematspaltung deutlich überlegen. Es ist besonders geeignet für die Gewinnung der enantiomerenreinen neutralen und basischen L-Aminosäuren, beispielsweise Lysin, Tryptophan, Histidin, Phenylalanin, Leucin, Isoleucin, Threonin, Methionin, Valin oder Arginin.

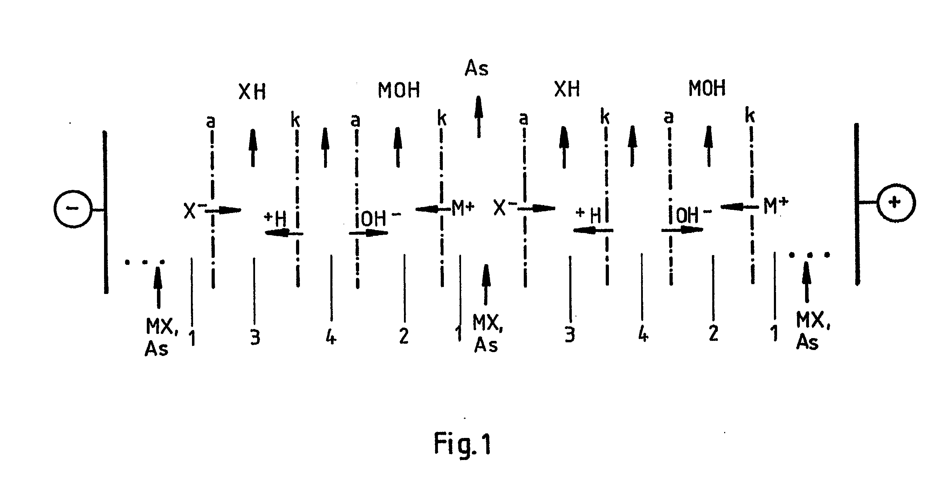

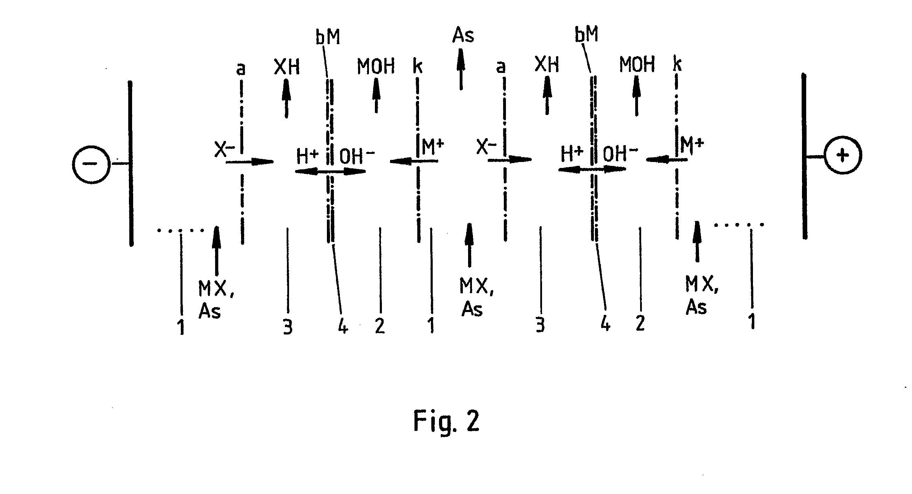

In den beigefügten Figuren 1 und 2 sind zwei Ausführungsformen der beim erfindungsgemäßen Verfahren zu verwendenden Elektrodialysevorrichtung schematisch dargestellt:

Figur 1 zeigt eine Anordnung, in der als Einrichtungen (4) zur Wasserzerlegung zusätzliche Kammern zwischen den jeweiligen 3-Kammer-Paketen vorgesehen sind. Es bedeuten 1 eine Speise- und Diluatkammer , 2 eine kathodenseitig davon angeordnete (basische) Konzentratkammer, 3 eine anodenseitig von der Speise- und Diluatkammer angeordnete (saure) Konzentratkammer, 4 eine zusätzliche Kammer zur Wasserzerlegung, k eine Kationenaustauschermembran, a eine Anionenaustauschermembran, As die Aminosäure, MX die Salze, MOH die aus den Kationen M⁺ entstehenden Hydroxide und XH die aus den Anionen X⁻ entstehenden Säuren.

Figur 2 zeigt eine Anordnung, in der als Einrichtungen (4) zur Wasserzerlegung sogenannte bipolare Membranen (bM) zwischen den jeweiligen 3-Kammer-Paketen vorgesehen sind. 4 bedeutet daher eine bipolare Membran, alle anderen Ziffern und Abkürzungen haben die gleiche Bedeutung wie in Figur 1.

Zur praktischen Durchführung des erfindungsgemäßen Verfahrens wird, sofern eine Vorrichtung der in Figur 1 dargestellten Art eingesetzt wird, in die als Einrichtungen (4) zur Wasserzerlegung dienenden zusätzlichen Kammern Wasser eingefüllt, das den Leitfähigkeitsvermittler enthält. Wird eine Vorrichtung der in Figur 2 dargestellten Art eingesetzt, entfällt diese Maßnahme. In die Anodenkammer wird 1N H₂SO₄ eingefüllt. Die Speise- und Diluatkammern (1) und die Konzentratkammern (2 und 3) werden in drei voneinander unabhängige Pumpkreisläufe zusammengefaßt, die jeweils ein Puffergefäß enthalten. In den Pumpenkreislauf für die Diluatkammern (1) wird die aufzuarbeitende Lösung aus der Racematspaltung umgepumpt, in dem Pumpenkreislauf für die kathodenseitig angeordne-

ten Konzentratkammern (2) Wasser, das zur Inbetriebnahme der Vorrichtung eine geringe Menge Natronlauge enthält, und in dem Pumpenkreislauf für die anodenseitig angeordneten Konzentratkammern (3) Wasser, das zur Inbetriebnahme der Vorrichtung eine geringe Menge Essigsäure enthält. In allen drei Pumpenkreisläufen wird die jeweils enthaltene Lösung im allgemeinen mit einer linearen Strömungsgeschwindigkeit zwischen etwa 0,5 und etwa 10 cm/sec umgepumpt. Die Vorrichtung wird mit Gleichstrom betrieben, die Stromdichte ist von den jeweiligen Konzentrationsverhältnissen abhängig und liegt im allgemeinen zweckmäßig zwischen 10 und 40 mA/cm².

In dem Pumpenkreislauf für die Speise- und Diluatkammern (1) wird der pH zweckmäßigerweise zwischen 4 und 8, vorzugsweise zwischen 5 und 7, gehalten, was erforderlichenfalls durch die Zugabe von Essigsäure oder Natronlauge bewerkstelligt werden kann. In dem Pumpenkreislauf für die kathodenseitig angeordneten Konzentratkammern (2) kann es zur Schonung der Anionenaustauschermembranen vorteilhaft sein, wenn der pH nach oben so begrenzt wird, daß er den Anionenaustauschermembranen gerade noch zuträglich ist. Der höchstzulässige pH hängt von der Art und den Eigenschaften der eingesetzten Anionenaustauschermembranen ab. Er kann dadurch eingehalten werden, daß die entstehende Lauge durch die Zugabe von fester N-Acetyl-DL-aminosäure zumindest teilweise neutralisiert wird. In diesem Falle wird dann aus diesem Kreislauf nicht reine Lauge, sondern ein Gemisch aus der Lauge und dem entsprechenden Salz der N-Acetyl-DL-aminosäure abgezogen. Da die Lauge ohnehin zum Auflösen von frischer N-Acetyl-DL-aminosäure dienen soll, kann auch das Gemisch problemlos vollständig recycliert werden.

Wenn in dem Pumpenkreislauf für die Speise- und Diluatkammern (1) der gewünschte Entsalzungsgrad erreicht ist, werden die Pumpen und der Strom abgestellt. Die drei Kreislauflösungen werden getrennt entnommen und in der bereits geschilderten Weise weiterverarbeitet.

Bei kontinuierlicher Arbeitsweise kann dem Pumpenkreislauf für die Speise- und Diluatkammern (1) soviel entsalzte Lösung entnommen werden, daß der Kreislauf gerade noch gewährleistet ist. Anschließend wird die Volumendifferenz durch Zugabe von noch nicht entsalzter Spaltlösung wieder ausgeglichen. Entsprechend kann auch in den Pumpenkreisläufen für die Konzentratkammern (2) und (3) verfahren werden. Es ist aber auch eine kontinuierliche "feed-and-bleed"-Arbeitsweise möglich.

Die Erfindung wird durch die nachfolgenden Beispiele näher erläutert:

Beispiel 1:

Es wurde eine Elektrodialysevorrichtung der in Figur 1 dargestellten Art eingesetzt. Es wurden drei voneinander unabhängige jeweils über Puffergefäße geführte Pumpenkreisläufe eingerichtet, in denen die jeweils parallel geschalteten Speise- und Diluatkammern ("Diluatkreislauf"), kathodenseitig angeordneten Konzentratkammern ("basischer Konzentratkreislauf") und anodenseitig angeordneten Konzentratkammern ("saurer Konzentratkreislauf") zusammengefaßt waren.

Die zusätzlichen, als Einrichtungen zur Wasserzerlegung dienenden Kammern wurden mit einer 2 gewichtsprozentigen Suspension eines Leitfähigkeitsrußes in Wasser beschickt und die Anodenkammer wurde mit 1N H$_2$SO$_4$ gefüllt.

Als Kationenaustauschermembranen fanden solche auf der Basis von Polystyrolsulfonsäure und als Ionenaustauschermembranen solche mit derivatisierten Pyridiniumendgruppen Verwendung.

In den Diluatkreislauf wurde eine Lösung folgender Zusammensetzung

32 g/l L-Methionin
92 g/l N-Acetyl-D(L)-methionin als Natriumsalz
38 g/l Natriumacetat,

in den basischen Konzentratkreislauf zum Anfahren Wasser mit einer geringen Menge an Natronlauge und in den sauren Konzentratkreislauf zum Anfahren Wasser mit einer geringen Menge Essigsäure eingefüllt.

Die drei Pumpen wurden in Gang gesetzt und auf eine lineare Strömungsgeschwindigkeit von etwa 1 cm/sec eingestellt. Es wurde ein Gleichstrom von i = 25 mA/cm² angelegt und es wurde solange elektrodialysiert, bis eine Elektrizitätsmenge von $8,3 \times 10^4$ coulomb pro Mol (0,86 Faraday pro Mol) anfänglich im Diluatkreislauf enthaltenem Anion geflossen war.

Im Diluatkreislauf befand sich nun eine Lösung folgender Zusammensetzung:

41 g/l L-Methionin
36 g/l N-Acetyl-D(L)-methionin als Natriumsalz
2 g/l Natriumacetat.

Die Lösung hatte einen pH von etwa 4,9, ihre Temperatur betrug etwa 30°C. Das Volumen hatte auf 78% des Anfangsvolumens abgenommen.

Im basischen Konzentratkreislauf wurde, um ein eventuell die Anionenaustauschermembranen schädigendes Ansteigen des pH auf über 8 zu verhindern, die entstandene Lauge immer wieder durch die Zugabe von festem N-Acetyl-DL-methionin teilweise neutralisiert, so daß eine Lösung mit ca. 115 g/l des Natriumsalzes von N-Acetyl-DL-methionin neben einer geringen Menge Natronlauge entstand.

Im sauren Konzentratkreislauf befand sich nach beendeter Elektrodialyse eine Lösung folgender Zusammensetzung:

130 g/l N-Acetyl-D(L)-methionin als freie Säure
38 g/l Essigsäure.

Die Lösung hatte einen pH von 1,95. Die Stromausbeute für die Überführung der Anionen betrug ca. 90%.

Die Lösung aus dem Diluatkreislauf wurde anschließend auf ca. 45% ihres Volumens eingedampft. Beim Abkühlen kristallisierte L-Methionin aus, das abgeschleudert und getrocknet wurde. Das abgeschleuderte Produkt besaß eine Reinheit von 99,7%, wies eine spezifische Drehung von $[\alpha]_D^{20} = 24°$ auf, ergab keine meßbare Sulfatasche und enthielt nur 0,1% N-Acetyl-D(L)-methionin und 0,2% D-Methionin als Verunreinigungen. Die Kristallisationsmutterlauge wurde recycliert und zusammen mit frischer Lösung aus der Racematspaltung erneut der Elektrodialyse unterworfen.

Die Lösung aus dem basischen Konzentratkreislauf wurde in die enzymatische Racematspaltung recycliert.

Die Lösung aus dem sauren Konzentratkreislauf wurde unter vermindertem Druck bis fast zur Trockne eingedampft. Dabei hinterblieb eine erstarrende Schmelze von N-Acetyl-D(L)-methionin mit ca. 0,5% restlicher Essigsäure. Nach anschließender Reracemisierung konnte auch dieses Produkt in die enzymatische Racematspaltung recycliert werden.

Beispiel 2:

Es wurde die gleiche Elektrodialysevorrichtung wie im Beispiel 1 eingesetzt.

Die zur Wasserzerlegung dienenden Kammern wurden mit einer Lösung von 15 Gewichtsprozent Polyacrylsäure in Wasser beschickt. Als die Kammern begrenzende Membranen wurden die gleichen Anionen- und Kationenaustauschermembranen verwendet, die im restlichen Membranstapel Verwendung fanden.

Die Entsalzung der gleichen Lösung wie im Beispiel 1 wurde unter sonst gleichen Bedingungen mit zwei Diluatkammern und insgesamt vier Konzentratkammern bei einer mittleren Zellspannung von ca. 40 V zum gleichen Ergebnis geführt. Es konnte kein Verlust von Polyacrylsäure in die Konzentratkammern nachgewiesen werden.

Beispiel 3:

Im gleichen Membranstapel wie in den vorangegangenen Beispielen wurde die wasserspaltende Kammer durch eine bipolare Membran ersetzt. Der Stapel enthielt nun drei Diluatkammern, insgesamt sechs Konzentrat- und zwei Elektrodenkammern.
In den Diluatkreislauf wurde eine Lösung mit folgender Zusammensetzung eingefüllt:

35 g/l L-Valin,
54 g/l N-Acetyl-D(L)-valin als Natriumsalz und
11 g/l Natriumacetat,

in die Konzentratkreisläufe wie im Beispiel 1 die entsprechenden Anfahrlösungen und in den Elektrodenkreislauf eine 0,5 M Na$_2$SO$_4$-Lösung.

Nach dem Ingangsetzen der Pumpen wurde Gleichstrom mit der Stromdichte i=25mA/cm$^2$ an die Zelle angelegt, wobei sich eine mittlere Zellspannung von 30 V einstellte. Nach dem Durchgang einer Elektrizitätsmenge von $8,88 \times 10^4$ coulomb pro Mol (0,92 Faraday pro Mol) anfänglich im Diluatkreislauf enthaltener Anionen hatte die entsalzte Lösung folgende Zusammensetzung:

41 g/l L-Valin,
22 g/l N-Acetyl-D(L)-valin als Natriumsalz und
0 g/l Natriumacetat.

Die Lösung hatte einen pH-Wert von etwa 5, ihre Temperatur betrug etwa 30°C.
Im sauren Konzentratkreislauf konnte eine Lösung mit

130 g/l N-Acetyl-D(L)-valin als freie Säure und
86 g/l Essigsäure

abgezogen werden.
Die Stromausbeute für die Überführung der Anionen betrug ca. 75 %.
Die Lösung aus dem Diluatkreislauf wurde auf ca. 40 % ihres Volumens eingedampft und abgekühlt. Durch Abschleudern konnte L-Valin mit einer Kristallisationsausbeute von 60 % isoliert werden.

Das kristalline Produkt wies einen Gehalt von 99%, einen Drehwert $[\alpha]_D^{25} = 27,4°$ und einen Aschegehalt von 0,01 % auf.

Die saure Konzentratlösung wurde wie im Beispiel 1 in die Reracemisierung rezirkuliert.

## Patentansprüche

1. Verfahren zur Aufarbeitung der nach Abtrennung des Enzyms verbleibenden Lösung aus der enzymatischen Racematspaltung einer N-Acetyl-DL-aminocarbonsäure in Gegenwart einer L-Aminosäureacylase durch Elektrodialyse, dadurch gekennzeichnet, daß die verwendete Elektrodialysevorrichtung aus einer Vielzahl von 3-Kammer-Paketen besteht, die ihrerseits jeweils eine Speise- und Diluatkammer (1), eine kathodenseitig angeordnete Konzentratkammer (2) und eine anodenseitig angeordnete Konzentratkammer (3) aufweisen, wobei jedes 3-Kammer-Paket von jedem der beiden benachbarten durch eine Einrichtung (4) getrennt ist, in der Wasser in OH$^-$-Ionen und H$^+$-Ionen zerlegt wird, und daß nach beendeter Elektrodialyse aus den Diluatkammern (1) eine wäßrige Lösung der L-Aminosäure und von höchstens 50% der ursprünglich vorhandenen Anionen in Form ihrer Salze, aus den kathodenseitig angeordneten Konzentratkammern (2) eine wäßrige Lösung der ursprünglich vorhandenen Kationen als Hydroxide und aus den anodenseitig angeordneten Konzentratkammern (3) eine wäßrige Lösung von N-Acetyl-D(L)-aminosäure und Essigsäure in Form der freien Säuren abgezogen wird.

2. Verfahren nach Anspruch 1, dadurch gekennzeichnet, daß die Einrichtungen (4) zur Wasserzerlegung aus einer zusätzlichen Kammer bestehen, die mit Wasser gefüllt ist, das einen Leitfähigkeitsvermittler enthält, für welchen die die Kammer begrenzenden Membranen undurchlässig sind.

3. Verfahren nach Anspruch 2, dadurch gekennzeichnet, daß der Leitfähigkeitsvermittler ein suspendiertes inertes leitfähiges Material ist.

4. Verfahren nach Anspruch 2, dadurch gekennzeichnet, daß der Leitfähigkeitsvermittler eine höhermolekulare Säure oder Base ist.

5. Verfahren nach Anspruch 1, dadurch gekennzeichnet, daß die Einrichtungen (4) zur Wasserzerlegung aus sogenannten bipolaren Membranen bestehen.

## Claims

1. A process for working up the solution -- remaining after separation of the enzyme -- from the enzymatic racemate resolution of an N-acetyl-DL-aminocarboxylic acid in the presence of an L-amino acid acylase by electrodialysis, characterized in that the electrodialysis unit used consists of a plurality of 3-chamber packs of which each in turn consists of a feed and diluate chamber (1), a concentrate chamber (2) on the cathode side and a concentrate chamber (3) on the anode side, each 3-chamber pack being separated from each of the two adjacent packs by a device (4) in which water is split into OH⁻ions and H⁺ ions and in that, on completion of electrodialysis, an aqueous solution of the L-amino acid and at most 50% of the anions originally present in the form of their salts is run off from the diluate chambers (1), an aqueous solution of the cations originally present in the form of hydroxides is run off from the concentrate chambers (2) on the cathode side and an aqueous solution of N-acetyl-D(L)-amino acid and acetic acid in the form of the free acids is run off from the concentrate chambers (3) on the anode side.

2. A process as claimed in claim 1, characterized in that the devices (4) for splitting water consist of an additional chamber which is filled with water containing a conductivity promoter to which the membranes confining the chambers are impermeable.

3. A process as claimed in claim 2, characterized in that the conductivity promoter is a suspended, inert, conductive material.

4. A process as claimed in claim 2 , characterized in that the conductivity promoter is an acid or base of relatively high molecular weight.

5. A process as claimed in claim 1, characterized in that the devices (4) for splitting water consist of so-called bipolar membranes.

## Revendications

1. Procédé pour le traitement par électrodialyse de la solution restant après séparation de l'enzyme provenant de dédoublement enzymatique de racémate d'un acide N-acétyl-DL-aminocarboxylique en présence d'une L-aminoacide acylase, caractérisé en ce que l'appareil d'électrodialyse utilisé est constitué de plusieurs groupes de 3 chambres qui, pour leur part, comportent chacun une chambre d'alimentation et de produit dilué (1), une chambre de concentré (2) disposée du côté de la cathode et une chambre de concentré (3) disposée du côté de l'anode, chaque groupe de 3 chambres étant séparé de chacun des deux voisins par un dispositif (4) dans lequel de l'eau est décomposée en ions OH⁻ et ions H⁺, et en ce que, une fois terminée l'électrodialyse, on évacue hors des chambres de produit dilué (1) une solution aqueuse du L-aminoacide et d'au plus 50% des anions, sous forme de leurs sels, qui étaient initialement présents, hors des chambres de concentré (2) disposées du côté de la cathode, une solution aqueuse des cations, sous forme d'hydroxydes, qui étaient initialement présents, et hors des chambres de concentré (3) disposées du côté de l'anode, une solution aqueuse de N-acétyl-D(L)-aminoacide et d'acide acétique, sous forme des acide libres.

2. Procédé selon la revendication 1, caractérisé en ce que les dispositifs (4) pour la décomposition de l'eau consistent en une chambre supplémentaire qui est remplie avec de l'eau contenant un agent augmentant la conductivité, que ne laissent pas passer les membranes limitant les chambres.

3. Procédé selon la revendication 2, caractérisé en ce que l'agent augmentant la conductivité est une substance conductrice inerte en suspension.

4. Procédé selon la revendication 2, caractérisé en ce que l'agent augmentant la conductivité est un acide ou une base à haut poids moléculaire.

5. Procédé selon la revendication 1, caractérisé en ce que les dispositifs (4) pour la décomposition de l'eau consistent en des membranes dites bipolaires.

Fig.1

EP 0 232 486 B1

Fig. 2